# EUROPEAN PATENT APPLICATION

(11) **EP 4 144 839 A1**
(43) Date of publication of application: **08.03.2023**
(21) Application number: 21382796.7
(22) Date of filing: 03.09.2021
(51) Int. Cl.: C12N 9/10, A61P 17/10, A61K 35/76, A61P 31/04, C12Q 1/70, C12N 7/00

(54) **A METHOD FOR SCREENING FOR MODIFICATIONS IN THE INFECTIVITY RANGE OF BACTERIOPHAGES DUE TO EPIGENETIC IMPRINTING**

(71) Applicant: Universitat Pompeu Fabra, 08002 Barcelona (ES)
(72) Inventor: GÜELL CARGOL, Marc, 08002 Barcelona (ES); KNÖDLSEDER, Nastassia Johanna, 08002 Barcelona (ES)
(74) Representative: Hoffmann Eitle

(57) **Abstract**

The present invention relates to a method for screening for changes in the infectivity range of one or more bacteriophages due to epigenetic imprinting of the bacteriophage(s) as well as a method for screening for one or more bacteriophages with a specific infectivity range due to the epigenetic imprinting of the bacteriophages. The invention furthermore relates to compositions comprising bacteriophages identified and obtained or obtainable by these methods and their use in therapy as well as for the selective killing of bacteria

## Description

### Technical field

The invention relates to methods for screening for modifications in the infectivity of bacteriophages that are due to epigenetic imprinting, compositions comprising bacteriophages obtained or obtainable by these methods and their use in therapy and for the selective killing of bacteria.

### Background of the invention

Phage therapy is a practice used to treat bacterial infections. The principle of this treatment is based on the use of a bacteriophage. Phages are non-living biological entities which consist of RNA or DNA and which can selectively kill bacteria depending on their host specificity. While phage therapy has already existed for almost a century its usage for therapy was seen controversial. Nowadays, phage therapy has widely been reconsidered as an alternative to the use of antibiotics to treat bacterial infections. Antibiotic treatment is often linked to the occurrence of antibiotic resistance within the pathogenic phenotypes but also kills beneficial non-pathogenic bacteria due to its non-selective nature. In contrast to antibiotics, phages can be highly specific in killing bacteria and kill selectively even on strain level.

The bacteriophages capable of selectively killing bacteria can either be isolated from the bacterial strain that shall be targeted itself, or they can be modified by genetic modification to specifically target the desired strain. One method of genetically modifying phages is through traditional homologous recombination-based techniques, such as phage crosses. A more targeted approach is the homologous recombination between the plasmid and phage genome generating recombinant phages with gene replacements, deletions, or insertions. Bacteriophage Recombineering of Electroporated DNA (BRED), CRISPR-Cas-Based Phage Engineering and Rebooting Phages Using Assembled Phage Genomic DNA are further known methods of genetically engineering phages (Chen et. al, 2019). Phage engineering provides one strategy to generate phage variants with unique properties, which might accelerate the development of phage therapy.

Another way of modifying a bacteriophage is to alter its epigenetic imprint. It is generally known in the art that growing, or culturing, a phage in different strains of bacteria can alter the properties of the phage. This alteration may be epigenetic, meaning that it is not due to changes in the nucleic acid sequence encoded by the genome of the phage, but instead appears to be due to chemical changes modifying the bases in the DNA. Changes include, for example, changes in the methylation of the nucleic acid in the phage due to DNA methylases.

Bacteria employ restriction-modification (R-M) systems to destroy invading DNA, while keeping the self-DNA safe by methylation of specific sites (Samson et al., 2013; Seed, 2015). Furi et. al. (2019) have shown that in *Streptococcus pneumoniae,* phase-variable type I restriction-modification (R-M) systems are part of the core genome and it was hypothesized that the ability of the R-M systems to switch between six target DNA specificities has a key role in preventing the spread of bacteriophages. However, phages can incorporate base modification systems to keep their genome resistant to the bacterial R-M systems (Samson et al., 2013). For example, T4 phage modifies the cytosines by two modifications, 5-hydroxymethylation and glucosylation, which make it highly resistant to virtually all the restriction endonucleases of E. *coli* (Bryson et al., 2015). MTases have been described in the prior art as a protective mechanism against bacteriophage infection that selectively transfer the methyl group from SAM to the nitrogen atoms at position 4 in cytosine and position 6 of adenine (m4C, m6A) or the fifth carbon of cytosine (m5C) within specific sequence motifs along the bacterial genome identified by the R-M system recognition domain (Vasu and Nagaraja (2013)). These methylated sequences are resistant to endonuclease digestion by the restriction enzyme and are recognized by the R-M system as a means of establishing self from nonself. Any phage DNA entering the host is assessed by the R-M system and digested by the R-M endonuclease if methylation is not detected by the corresponding recognition domain. To circumvent host restriction of phage DNA, bacteriophages often introduces their own MTases during infection.

The endless coevolution of phages and their host bacteria makes bacteria less resistant to phage therapy than for example the antibiotic treatment, especially when phage cocktails are used.

For example, WO2009087356A1 discloses that culturing phages in *Staphylococcus aureus* can be used to increase the host range of *Staphylococcus aureus* strains that can be infected by a phage. This reduces the number of different phages that need to be included in a panel of phages to ensure a broad coverage of different bacterial strains. This heterogenous panel of phages can then be used for example in disinfectant compositions for surfaces as it provides a mix of phages capable of killing many different bacteria.
*Cutibacterium acnes* (C. *acnes*)*,* also known as *Propionibacterium acnes* (*P. acnes*) is the major skin commensal of the human skin microbiome but has been associated with the skin condition acne vulgaris. C. *acnes* harbors multiple lytic or temperate phages. Phages within the skin modulate skin microbiome composition by infecting either a narrow range of strains or broadly within a population. It has been shown that healthy skin harbors more phages than acne/unhealthy skin. C. *acnes* is resistant to multiple antibiotics either directly or by the formation of a stable biofilm which can protect bacteria from antibiotic activity. However, phage therapy can provide a suitable therapy because it has been shown that phages can dissolve biofilms. WO2019113066A1 discloses a bacteriophage treatment for acne where lytic bacteriophages are capable of lysing P. *acnes* thus preventing the formation of biofilms and acne.

However, due to the fact that C. *acnes* is at the same time the major component of healthy skin its concrete involvement in the disease is not fully understood. C. *acnes* contains various strains, some of which are beneficial to the skin microbiome, such as strain H1 (clade IB), whilst others, such as strain A1 (clade IA1), are associated with pathogenic states (Dreno B. et al. 2018). It would therefore be useful when treating skin to be able to distinguish between the beneficial and the pathogenic strains of C. *acnes* in order to provide for a healthy microbiome of the skin.

To date none of the available phage therapies provide a possibility to target only certain strains of C. *acnes,* while not affecting other beneficial probiotic strains. There is thus a need for a method for obtaining a selective and specific phage population that is targeted to the killing of preferably only one specific strain of C. *acnes* while not harming other probiotic strains.

Other applications where bacteriophage therapy is an interesting alternative to the use of antibiotics include targeting Methicillin-resistant *Staplylococcus aureus* (MRSA) that can cause systemic infections in sick, elderly or immune-compromised patients as well as other bacteria increasingly causing problems in hospitals, such as *Clostridium difficile* or *Pseudomonas aeruginosa.* Especially, since mutations confer antibiotic resistance, or genes encoding antibiotic resistance enzymes, such as penicillinases, are becoming increasingly common in pathogenic bacteria world-wide, there is a great need for a different approach in fighting bacteria.
With the large amount of possible applications of bacteriophages against all kinds of pathogenic bacteria, there is a need for a simple, rapid, economic and efficient method of screening for bacteriophages with a desired infectivity range allowing to identify and isolate the bacteriophages that have obtained the desired specific properties without the need of applying lengthy genetical modification to the phages as have been abundantly described in the prior art.

### Summary of the Invention

The present invention therefore relates to an *in vivo* or *in vitro* method for screening for changes in the infectivity range of one or more bacteriophages due to epigenetic imprinting of the bacteriophage(s), the method comprising the following steps:
(a) obtaining an isolate of the one or more bacteriophages after infection of at least a bacterium with said one or more bacteriophages, said bacterium preferably comprising a restriction methylation system; and
(b) infecting a bacterium different from the bacterium used in step a), said bacterium preferably comprising a restriction methylation system, with the isolate of the one or more bacteriophages that previously infected the bacterium of a) in order to allow multiplication of said phage; and
(c) obtaining an isolate of the one or more bacteriophages after infection of step b), and determining the changes in the infectivity range of the one or more bacteriophages due to the epigenetic imprinting of the bacteriophage(s) after the infection of the bacterium of step b); and optionally
(d) selecting said isolate of the one or more bacteriophages if the sought-after specific infectivity range due to the change in the epigenetic imprinting is obtained and, optionally, formulating said bacteriophage(s) into a product.

In a further embodiment the method comprises characterizing the epigenetic imprinting of the isolates of step c) and/or d).

The present invention furthermore relates to an *in vivo* or *in vitro* method for screening for one or more bacteriophages with a specific infectivity range due to the epigenetic imprinting of the bacteriophage(s), the method comprising the following steps:
(a) obtaining an isolate of the one or more bacteriophages after infection of at least a bacterium with said one or more bacteriophages, said bacterium preferably comprising a restriction methylation system; and
(b) infecting a bacterium different from a), said bacterium preferably comprising a restriction methylation system, with the one or more bacteriophages that previously infected the bacterium of a) in order to allow multiplication of said phage; and
(c) obtaining an isolate of the one or more bacteriophages after infection of step b), and determining whether the one or more bacteriophages have the specific infectivity range due to the epigenetic imprinting of the bacteriophage(s) after the infection of the bacterium of step b), and
(d) selecting said isolate of the one or more bacteriophage if the sought-after specific infectivity range due to the change in the epigenetic imprinting is obtained and, optionally, formulating said bacteriophage(s) into a product.

In a further embodiment of the method of present invention the bacterium of step b) comprises a restriction methylation system.

In a further embodiment of the method of present invention, the isolate of bacteriophages after infection of step b) comprises a substantially homogenous population of bacteriophages with a changed infectivity range due to the epigenetic imprinting.

In one embodiment the bacterium of a) and b) is selected from the group consisting of: C. *acnes, E.coli,* Salmonella species, such as *Salmonella typhimurium,* Shigella species, such as *Shigella flexneri,* Enterococcus species, such as *Klebsiella,* Proteobacteria species, such as Pseudomonas species, specifically *Pseudomonas aeruginosa,* Haemophilus species, Firmicutes species, such as Bacillus species, Streptococcus and Staphylococcus, specifically *Staphylococcus aureus.* In a preferred embodiment the bacterium is C. *acnes.*

In one embodiment the bacterium of a) and b) of the methods described above are different strains of C. *acnes,* the bacterium of step b) being characterized by comprising an active restriction methylation system such as a type Ig, type Ila, type IIb, type IIg or type IIb restriction methylation system.

In a further embodiment the bacterium of a) and b) is a different strain of C. *acnes* and each of said strains pertains to any of the following clades IA1, IA2, IB, IC, type II or type III.

In one embodiment the bacterium of a) pertains to the IA1, IA2 or IB clade and the bacterium of b) pertains to any of the following clades IA1 or IA2, IB, IC, type II or type III.
In another embodiment the bacterium of a) pertains to the IA1, IA2 or IB clade and the bacterium of b) pertains to any of the following clades IB, IC, type II or type III.
In one embodiment the present invention relates to a composition comprising a phage population as identified and obtained or obtainable by the methods as described above.

In a further embodiment the present invention relates to the composition comprising a phage population as identified and obtained or obtainable by the methods as described above for use in therapy.

In one embodiment the present invention relates to the composition comprising a phage population as identified and obtained or obtainable by the methods as described above for use in the selective bacterial killing of one or more bacterial species or strains preferably selected from any species or strains from the group consisting of C. *acnes, E. coli,* Salmonella species, such as *Salmonella typhimurium,* Shigella species, such as *Shigella flexneri,* Enterococcus species, such as *Klebsiella,* Proteobacteria species, such as Pseudomonas species, specifically *Pseudomonas aeruginosa.* Haemophilus species, and Firmicutes species, such as Bacillus species, Streptococcus and Staphylococcus, specifically *Staphylococcus aureus.*

In a further embodiment the present invention relates to the composition comprising a phage obtained or obtainable by the methods as described above for use in the selective bacterial killing of the one or more bacterial species or strains to enhance engraftment of probiotic strains.

In a further embodiment the present invention relates to the use, preferably the *in vitro* use, of the epigenetic imprint which is changed during phage reinfection of a suitable bacterium with a restriction methylation system to select phages capable of lysing and/or killing one or more bacterial species or strains and to obtain a substantially homogenous population of phages with a specific infectivity range.

### BRIEF DESCRIPTION OF THE FIGURES

Figure 1: Heat map of PAD20 phage infection properties on different C. *acnes* strains depending on its origin of extraction. SLST strain A1 is infected by all three PAD20 phage extractions. It was observed that H2 could not be infected by A1 originally while the H2 R-M KO strain shows a similar infection pattern to strain A1.
Figure 2: *C*. *acnes* H2 (also referred to as KPA171202) has a methylation motif in the AGCAGY sequence
   A) Fraction of methylated reads across the positions of the AGCAGY motif for the wild-type (in black) and the methyltransferase knock-out (KO) H2 (in grey) strains.
   B) Fraction of methylated reads in the methylated adenine at 4th position of the AGCAGY motif detected in the pMW535 plasmid after recombinant expression of the indicated methyltransferase originating from H1 or H2 C. *acnes* in a methylation deficient *E. coli* strain. Negative is the methylation deficient *E. coli* not being cotransformed with any methyltransferase of C. acnes.
   C) Fraction of methylated reads in the methylated adenine at 4th position of the AGCAGY motif in the PAD20 phage genome. Samples are named first after the infected strain where the phage was sequenced and second after the strain from which the phage was produced.(i.e, H2-H1 means a phage produced in H1 was used to infect H2 and then extracted for sequencing from H2) Significance was tested using ANOVA analysis (***, p < 0.001)

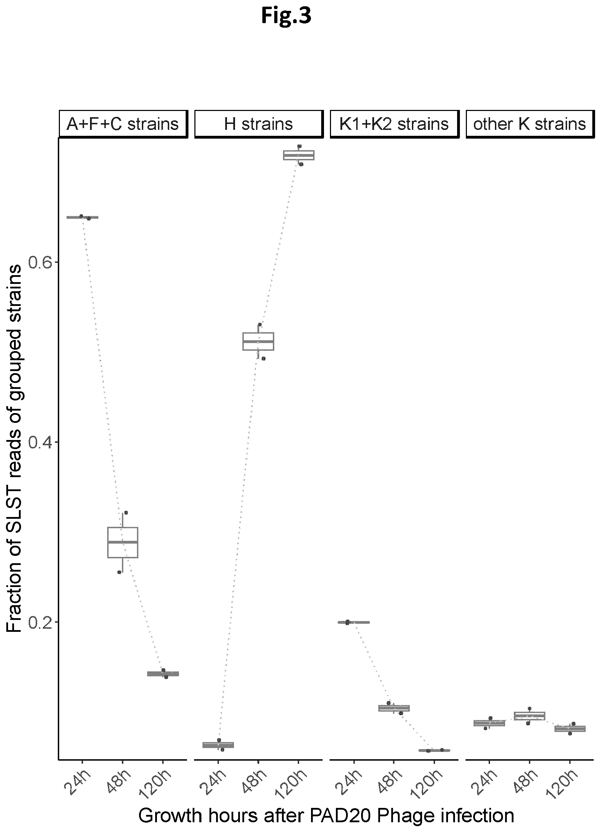

Figure 3: Selected killing of a population of strains in a whole microbiome sample infected with PAD20 originated from A1 after 3 different time points 24, 48, 120 h of growth. Distribution of proportions of sequences corresponding to each cluster of strains taking into account similarity to SLST alleles in the classification. Two replicates for each time point were made.

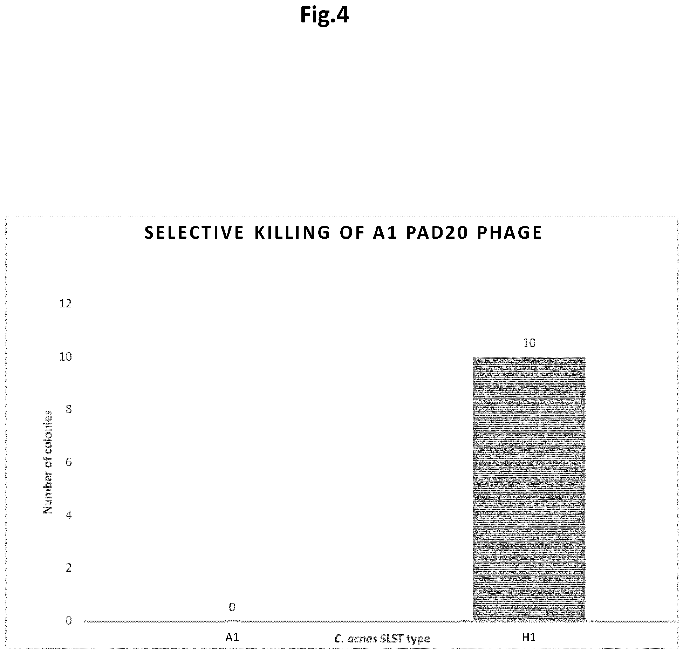

Figure 4: Selective killing of a mixed population of SLST strain A1 and H1 by PAD20 Phage extracted from SLST A1. After 3 days serial dilutions were plated, and 10 representative colonies screened for their SLST type. All colonies showed to be of SLST H1.

### Detailed description

The present invention may be understood more readily by reference to the following detailed description of the preferred embodiments of the invention, and to the examples included therein.

The present invention relates to an *in vivo* and *in vitro* method for screening for changes in the infectivity range of one or more bacteriophages due to epigenetic imprinting of the bacteriophage(s) as well as an *in vivo* and *in vitro* method for screening for one or more bacteriophages with a specific infectivity range due to the epigenetic imprinting of the bacteriophages. The invention furthermore relates to a composition comprising bacteriophages identified and obtained or obtainable by these methods. The invention furthermore relates to such compositions for use in therapy. The invention also relates to such compositions for use in selective killing of bacteria.

The inventors herein provide for a method to obtain an isolate of phages with a modified epigenetic imprinting. The epigenetic profiles of the phages can be adapted to program killing to specific subsets of bacteria. In contrast to the genetic engineering techniques used and described in the prior art for genetically modifying phages, such as for example homologous recombination-based techniques, Bacteriophage Recombineering of Electroporated DNA (BRED), CRISPR-Cas-Based Phage Engineering and Rebooting Phages Using Assembled Phage Genomic DNA, the bacteriophages used in present invention do not need to be subjected to such techniques in order to provide them with the desired specificity. Instead, the bacteriophages are modified by altering their epigenetic imprinting.

*Cutibacterium acnes* (C. *acnes*) has been used as a model organism by the inventors to develop the methods of present invention. It is, however, to be understood that these methods are not limited to C. *acnes* but can be applied to any desired bacterial species. Particularly suitable are all bacteria comprising a restriction methylation system.

While C. *acnes* phages have an overall sequence identity of more than 85%, their ability of infecting certain strains is very diverse. Since the reason for this diversity was not well understood, the inventors investigated other factors involved in host selectivity. Restriction methylation (R-M) is a host mechanism that protects the bacterial host from foreign incoming DNA. This restriction methylation can be detected by nanopore sequencing. The present invention takes into account epigenetic changes which can occur on the phage genome when injecting its DNA into the host and change the scope of a single phage to infect one additional specific strain of C. *acnes* allowing to specifically erase pathogenic strains and sustain probiotic ones.

In particular, the C. *acnes* bacteriophage named PAD20 extracted from AD20 which was found in a deep tissue infection and belongs to type II C. *acnes* strains was investigated (Lood et. al 2008). Since all C. *acnes* phages are very similar in protein pattern, morphology and coding genes the phages are classified depending on their host range. PAD20 was classified into the PA I group. As shown in Example 2, different C. *acnes* strains belonging to different classes of C. *acnes* like type IA1, IA2, IB, type II and type III were infected with C. *acnes* bacteriophage PAD20, which was previously extracted from either a type IA1 or IB strain of C. *acnes.* It was observed that the lysis properties differed from strain to strain depending on which host the phage was first isolated from (see Table 2, Example 2). While the phage extracted from a type IA1 strain like A1 was unable to infect a type IB strain (H2), it was able to infect other type IA1 or IA2 strains and H1. On the other hand, PAD20 extracted from a type IB strain was able to kill A1 and also itself but was unable to do so to H1. PAD20 from H1 on the other hand could infect all three classes and strains A1, H1 and H2. Strains belonging to type II strains were unable to be lysed by any of the extracted PAD20 phages.

Then, the inventors investigated differences in the host and saw differences in active restriction methylation systems. One system in KPA171202 (also called "H2") which belongs to type III R-M was found and its activity verified by nanopore sequencing. To understand the involvement of R-M in host range specificity of C. *acnes* phage PAD20, a knockout (KO) mutant in KPA171202 (H2) was created as described in detail in Example 3. This strain did not have the corresponding R-M system anymore and was unable to methylate its own DNA in a specific pattern.

After infection of this strain with PAD20 extracted from either H1, A1 and H2 infectivity of all three phages was observed confirming the hypothesis that epigenetic changes and specifically the R-M system is involved in phage host range specificity and that epigenetic profiles of the phages can be adapted to program killing to specific subsets of bacteria.

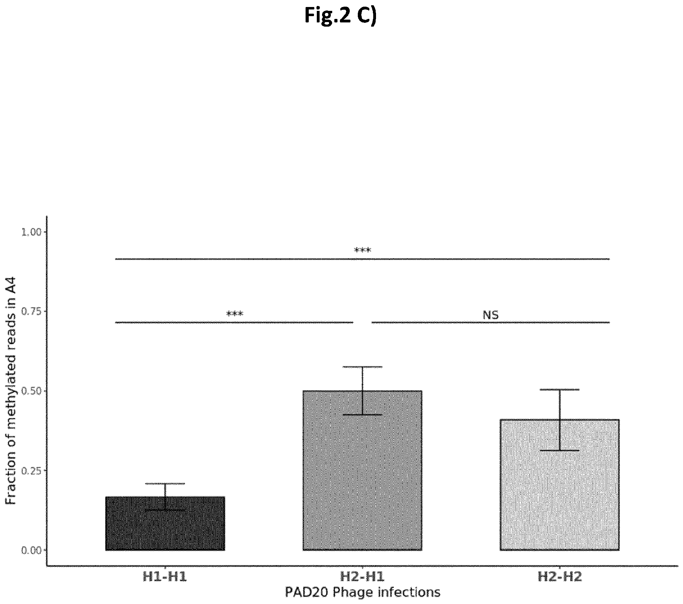

To understand if C. *acnes* H2 is methylating phage DNA during infection the inventors extracted phages isolated from H2 and also infected H2 with H1 PAD20 phage. They verified methylation of phage DNA by nanopore sequencing and identified the specific methylation pattern (Figure 2C).

To verify whether the isolated phages target a certain subpopulation only, the inventors have used a whole microbiome sample and infected it with PAD20 originating from A1. The complex microbiome sample contains many different strains and it was analyzed over time (24h, 48h and 120h) to see the dynamics of phage infection. While a decrease in SLST types belonging to A, F, C strains could be observed an increase in H strains was seen. In the case of K strains a difference was observed between K1 and K2 where the population decreases while other K strains are not affected by the phage (see Fig.3). This difference might indicate functional or unfunctional CRISR type I-E system in the K strains. This experiment showed that Clade IA1 and IA2 bacteria (SLST types A, F, C) were targeted by using a phage which is not methylated and probiotic strains such as H1 and H2 were preserved. The abundance of IA1 and IA2 reduced after time while IB strains increased.

To test specific killing of a population comprising two different C. *acnes* strains, the authors inoculated 1:1 a strain from subgroup IA1 (A1) with one of subgroup IB (H1) and infected those with a phage extracted from A1 host. After 3 days the inventors plated a dilution of bacteria on agar plates and they checked a subset for their classification by SLST sequencing. They verified that all tested colonies belonged to H1 C. *acnes* strain which meant a 100% host range specificity of PAD20 phage (see Fig.4).

From the results it can be deducted that this phage can be targeted to the killing of only one species of bacteria and contribute to a healthy microbiome without the use of antibiotics.

Some C. *acnes* strains, such as H1, are beneficial, while others are more associated with pathogenic states, such as A1. The inventors have shown in the past that it is possible to engraft probiotic strains, such as H1 (Paetzold et al. (2019)). In this sense, it is possible to enhance the engraftment of probiotic strains, such as H1, by clearing the ecological niche using phages as prepared by the present invention. In other words, applying the probiotic strain H1 together with phages programmed to lyse/kill the more pathogenic strain A1, which at the same time is inactive against H1, engraftment can be significantly enhanced.

Therefore, the inventors have shown the use of phages for the selective bacterial killing based on epigenetic imprints and the use of phages to enhance engraftment of probiotic strains, wherein such engraftment is enhanced by the selective bacterial killing based on epigenetic imprints.

The present invention therefore provides for a method to use epigenetic imprinting which occurs on the phage genome when injecting its DNA into the host to target the scope of a single phage to infect a specific C. *acnes* strain providing a possibility to erase pathogenic strains and sustain probiotic ones.

It is finally contemplated that any features described herein can optionally be combined with any of the embodiments of any method, medical uses and compositions of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application was specifically and individually indicated to be incorporated by reference.
The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

As used herein, the term "bacteriophage" or "phage" refers to a functional phage particle comprising a nucleic acid genome packaged in a proteinaceous envelope or capsid. The term also refers to portions of the bacteriophage, including, e.g., a head portion, or an assembly of phage components, which provide substantially the same functional activity.

The term "lysis" as used in the invention refers to the lytic activity of a bacteriophage causing the breaking down of the bacterial cell compromising its integrity. The term "killing" of the bacteria is equally used herein and can mean that the bacterium is killed due to the lytic activity of the bacteriophage. The lytic activity of a bacteriophage can be tested on for example C. *acnes* according to techniques known per se in the art (see also experimental section).

The term "specific" or "specificity" in relation to a bacteriophage refers to the type of host that said bacteriophage is able to infect, lyse and/or kill. A bacteriophage "specific" for a C. *acnes* strain refers to a bacteriophage which can infect, lyse and/or kill one specific C. *acnes* strain. A bacteriophage "specific" to C. *acnes* is a phage that is able to infect, lyse and/or kill only C. *acnes* and which essentially does not infect bacteria other than C. *acnes* under physiological conditions.

In the context of the present specification, the term "isolated bacteriophage" or "isolate of the one or more bacteriophages" should be considered to mean one or more bacteriophage(s) that is/are removed from its/their natural environment and/or separated from a component of its natural environment, such as for example its host bacterium.

The term "substantially homogenous population of phages" or "substantially homogenous population of bacteriophages" refers to a bacteriophage population with a high number of phages having the same infectivity patterns due to their epigenetic imprints as produced by the method of present invention, the number being high enough to confer the desired specificity for a certain strain. In a preferred embodiment of the invention the substantially homogenous population comprises at least 70%, 75%, 85%, 86%, 87%, 88%, 89%, 90%, 91%, 92%, 93%, 94%, 95%, 96%, 97%, 98%, 99% or 100% of phages having the same infectivity patterns due to their epigenetic imprints as produced by the method of present invention.

### EXAMPLES

### Example 1: a) Homology-based search of potential restriction methylation systems using REBASE, b) Genomic DNA extraction c) Bacterial lysates preparation and d) C. acnes lysate assay with plasmid DNA

### a) Homology-based search of potential restriction methylation systems using REBASE

In order to identify the potential R-M systems present in C. *acnes,* all the predicted ORFs corresponding to either methyltransferases or restriction enzymes from those strains for which complete genome assembly was available were retrieved from the REBASE database. For the strains with no information available, their respective genomes from the NCBI Genome database and performed protein-protein BLAST with the annotated coding sequences to identify the potential R-M homologs were retrieved. To further assess the difference between systems across strains, pairwise comparisons between ORFs belonging to the same putative system type by sequence alignment using the Clustal Omega algorithm available at EMBL-EBI portal were performed (Madeira et al. 2019). A complete list of strains and accession numbers used in this study can be found in Table 1 below:

**Table 1: List of strains and accession numbers used in this study**

| **Strain designation** | **SLST type** | **GeneBank Accession number** | **Notes** |
|---|---|---|---|
| 6609 | H1 | NC_017535.1 | |
| KCOM1315 | H1 | NZ_CP031442.1 | |
| KPA171202 | H2 | NC_006085.1 | used in this study |
| PA_21_1_L1 | H1 | NZ_CPOI23S1.1 | |
| PA_30_2_L1 | D1 | NZ_CP012350.1 | |
| 09-9 | K1 | LKVB00000000.1 | used in this study |
| ATCC11S28 | K9 | NC_017550.1 | |
| KCOM1861 | K2 | NZ_CP012647.1 | |
| P.acnes17 | F5 | NC_016512.1 | |
| P.acnes31 | F4 | NC_016511.1 | |
| P.acnes33 | F1 | NC_016516.1 | |
| P.acnes 266 | A1 | NC_017534.1 | |
| ATCC6919 | A1 | NZ_CP023676.1 | |
| FDAARGOS 503 | A1 | NZ_CP033842.1 | |
| FDAARGOS 577 | C1 | NZ_CP033718.1 | |
| Hdn-1 | A1 | NZ_CMO6O32.1 | |
| HL096PA1 | C1 | NC_021085.1 | |
| PA_12.1.L1 | A1 | NZ_CP012354.1 | |
| PA_12.1.R1 | A1 | NZ_CP012353.1 | |
| PA_15.1.R1 | C1 | NZ_CP012355.1 | |
| PA_15.2.L1 | A1 | NZ_CP012352.1 | |
| SK137 | C1 | NC_014039.1 | |
| TP-CU 389 | F1 | NZ_AP019664.1 | |
| Laboratory Strain H1 | H1 | | used in this study |
| Laboratory Strain A1 | A1 | | used in this study |
| Laboratory Strain K2 | K2 | | used in this study |
| Laboratory Strain A1 | A1 | | used in this study |
| Laboratory Strain C1 | C1 | | used in this study |

### b) Genomic DNA extraction

C. *acnes* SLST types A1, C1, H1, H2, K1, K2 were grown on Brucella agar plates for 3 days. Bacteria were collected with a sterile cotton swab and inoculated in 1 mL BHI media, spun down 3 min 8000xg and resuspended in 1 mL lysis buffer (9,4 parts TE buffer pH 8,0 and 0,6 parts 10% SDS). All content was transferred to tubes prefilled with 0.1 mm silica beads and lysed in a mechanical homogenizer (Savant Bio 101 FastPrep FP120 Cell disruption system) for 25 sec at 6,5 speed. Samples were placed on ice for 1 min and the cycle was repeated once more. Next, 6 ul Proteinase K (20 mg/mL) and 5 ul RNAse (1 mg/mL) were added and incubated 1 h at 37 °C water bath. Equal volume Phenol/Chloroform was added, mixed and spun down at max speed for 5 min. Then, the aqueous phase was collected and repeated the previous step. Equal volume of chloroform was added, mixed and spun down at max speed for 5 min. The aqueous phase was collected and 2,5 volumes 100 % ice-cold EtOH was added to precipitate DNA. Samples were kept for at least 1 h at -20 °C. Then, they were spun down at max speed for 15 min at 4°C. Supernatant was discarded and the pellet was washed with 70 °C EtOH. After, the sample was spun down for 2 min at max speed. Once dried, the pellet was resuspended in 50 ul H20 and DNA concentration was measured using Qubit high sensitivity DNA kit (Thermo Fisher).

### c) Bacterial lysates preparation

C. *acnes* strains were pre-cultured in 20 ml BHI broth with 180 rpm shaking at 37°C during 24 hours under anaerobic conditions. Then, they were re-inoculated in 1 L of fresh BHI with 1200 rpm of stirring, maintaining the same conditions. After 72h, cells were harvested by centrifugation at 5000 xg for 20 minutes and bacterial pellet was washed twice with 20 ml of cold K2PO4 50mM, pH 7.4. Then, 0.33 ml of S30 buffer (10 mM Tris(CH3COO) (pH 8.2), 14 mM Mg(CH3COO)2, 10 mM K(CH3COO) and 4 mM DTT ) were added per gram of wet cell mass and the suspension was transferred into 2ml microtubes prefilled with 0.1 mm glass beads. Using a mechanical homogenizer (Savant Bio 101 FastPrep FP120 Cell disruption system), cells were lysed following 2 cycles at 6000 rpm for 30 seconds. Samples were then filtered by centrifugation at 1000 xg for 5 minutes at 4°C using a 0.22 um filter unit. Then, the follow-through was centrifuged at 12.000 xg for 12 minutes at 4°C. Finally, clear supernatants were collected and stored at -80°C until they were used.

### d) C. acnes lysate assay with plasmid DNA

C. *acnes* lysates were incubated with 0.5 ug plasmid DNA together with different buffers (NEB) supplemented with or without ATP or SAM. Cutsmart (NEB) buffer was chosen for further experiments. 8 ul of C. acnes concentrated lysate was mixed with 2 ul of Cutsmart buffer (1x final) and 0,5 ug of plasmid DNA was added. Final volume was adjusted to 20 ul and samples were incubated for 4 h at 37 °C. Afterwards, DNA was observed on a 1 % agarose gel.

### Example 2: Infection of C. acnes strains type IA1, IA2, IB, type II and type III with C. acnes bacteriophage PAD20

C. *acnes* SLST types A1, H1 and H2 (KPA171202) (for strain KPA171202 see Brüggemann et al. (2004)) were infected with C. *acnes* PAD20 phage extracted from either A1 or H1. Brucella top agar plates (0,6 %) were prepared by mixing 4,5 mL Brucella agar with 0,5 mL Bacterial culture. After being solidified, phages were added in 10 ul drops and plates were incubated at 37 °C anaerobically for 3-5 days. After the first infection cycle, phages were extracted by adding 10 mL SM buffer (100 mM NaCl, 8 mM MgSO4, 50 mM Tris-HCI pH 7.5, 0.01% gelatin) to the plates and incubating 30 min at RT carefully rotating. Then SM-buffer was collected and NaCl was added to a final concentration of 0.5 M to release phages bound to bacterial debris. Samples were incubated for 30 min at RT and centrifuged at 8000g for 20 min. Then, samples were filtered and 10% (w/v) PEG 6000 was added. After 10 minutes incubation, the phages were collected by centrifugation (10,000 xg, 30 min). Finally, the pellet was resuspended in 300ul of SM buffer. These phages were used to re-infect C. *acnes* A1, A5, C1, C3, D1, F4, H1, H2 (KPA171202), K2 and L1 which can be categorized in different types IA1, IA2, IB, type II and type III.

As can be seen in Table 2 below, different lysis properties depending on which host the phage strain was extracted from could be observed. While the phage extracted from a type IA1 strain like A1 was unable to infect KPA (short for "KPA171202" also called "H2"), which belongs to type IB, it was able to infect other type IA 1 or 2 strains and H1. PAD20 extracted from KPA was able to kill A1 and also itself but was unable to do so to H1. PAD20 from H1 on the other hand could infect all three classes and strains A1, H1 and KPA. Strains belonging to type II strains were unable to be lysed by any of the extracted PAD20 phages. This could be due to the availability of functional CRISPR Cas type I-E systems which includes spacers against PAD20 phages.

**Table 2: Infectivity properties of phages extracted from A1, H1 or KPA (H2)**

| **Bacteria infected** | **A1 Phage** | **H1 Phage** | **KPA Phage** | **Clade** |
|---|---|---|---|---|
| A1 | +++ | +++ turbid | +++ | IA1 |
| H1 | + | +++ | - | IB |
| KPA | - | + | +++ | IB |
| D1 | turbid lysis | ++ turbid | - | IA1 |
| C1 | +++ | +++ | ++ | IA1 |
| C3 | +++ | +++ | +++ | IA1 |
| F4 | +++ | +++ | +++ | IA2 |
| K2 | - | - | - | II |
| L1 | full lysis | +++ | full lysis | III |
| A5 | +++ | +++ | ++ | IA1 |

### Example 3: Activity of the restriction methylation system

Differences in the activity of the type III restriction methylation system of the host KPA171202 were investigated by nanopore sequencing.

First, a methylation deficient KPA171202 mutant (KO mutant) was created following the method previously described by Sorensen et al. 2010. Shortly, 500 bp upstream (primers 468/469) and downstream (primers 470/471) of the locus to be replaced were amplified and cloned into the pGEM-T-easy vector. In a second step the erythromycin cassette was cloned between the homology arms using Acc65l restriction sites and positive clones selected to be transformed into dam-GM2199 cells. C. *acnes* competent cells were prepared as previously described (Sorensen et al. 2010).

Correct knockouts were verified by junction PCR and WGS followed by a functional characterization using nanopore sequencing and phage infection assay. Nanopore sequencing verified the loss of methylated AGCAGY motifs in comparison to the wildtype genome.

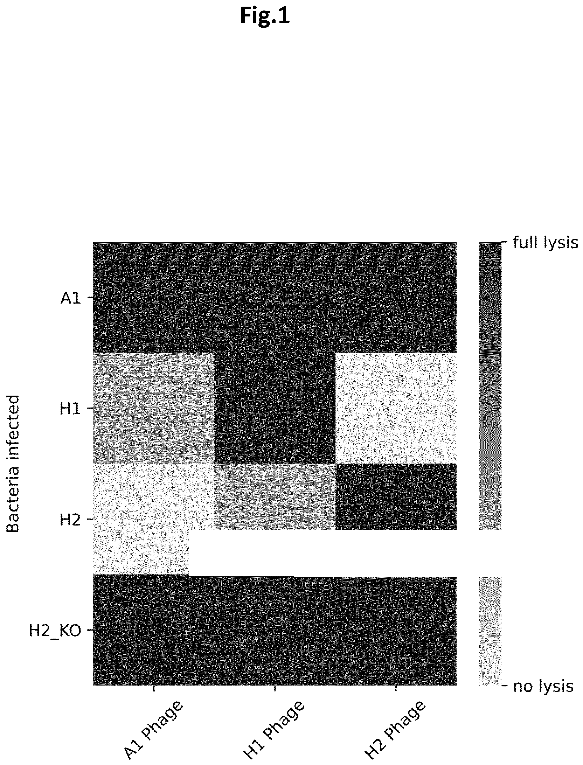

The Nanopore libraries were prepared as follows: Genomic libraries corresponding to C. *acnes* A1, H1, H2, C1, K1 and K2 strains, PAD20 phage DNA and E. coli plasmids were prepared using the Ligation Sequencing Kit SQK-LSK109 (Oxford Nanopore) according to the manufacturer's instructions. Native and WGA samples were barcoded using the Native Barcoding Kit (EXP-NBD104) and PCR Barcoding kit (EXP-PBC001), respectively. All samples were sequenced on the MinION platform either using a FLO-MIN106D (R9) flow cell or a FLO-FLG001 Flongle (R9.4.1) flow cell. Basecalling and demultiplexing were performed using Guppy v4.0.11. Nanopore sequencing data for the mentioned strain were analyzed using Tombo v1.5 software (Stoiber et al., n.d.) for detection of modified bases. In brief, current signals were compared against the KPA171202 reference genome using the "resquiggle" command. Then, methylation peaks were detected comparing the native sample and the whole genome amplification (WGA) sample for each strain using the "detect_modifications model_sample_compare" command. For the detection of methylated motifs, the 50 bp context of 1000 positions with the highest modified fraction (i.e. the fraction of reads identified as potentially methylated over all reads mapping to the given position in the genome) were extracted using the "text_output signif_sequence_context" command. This set of sequences was processed using MEME (Bailey et al. 2015) with 'zoops' mode to identify common sequences. Statistically significant modified fraction values for each genomic position were exported from tombo using tombo's "text_output browser_files" and were further analysed using a custom R script (see code availability). A similar procedure was used to analyse the Phage DNA and plasmid samples. For phage DNA, the genome reference was assembled de novo using Fyle (Kolmogorov et al. 2019) with the default parameters. Fine typing of the methylation motif was performed using the multi-label classification algorithm present in the Nanodisco (Tourancheau et al. 2021) toolkit according to its documentation.

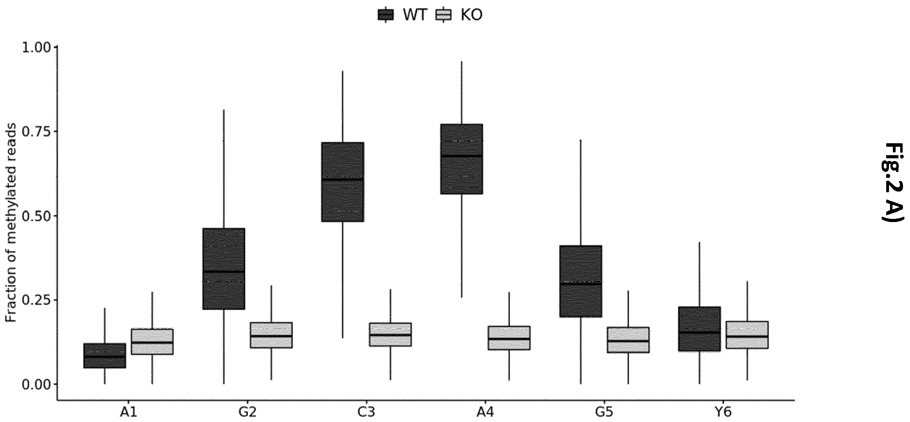

As shown in Figure 2A) the KO mutants (methylation deficient KPA171202) did not have the corresponding restriction methylation system anymore and were therefore unable to methylate its own DNA in a specific pattern.

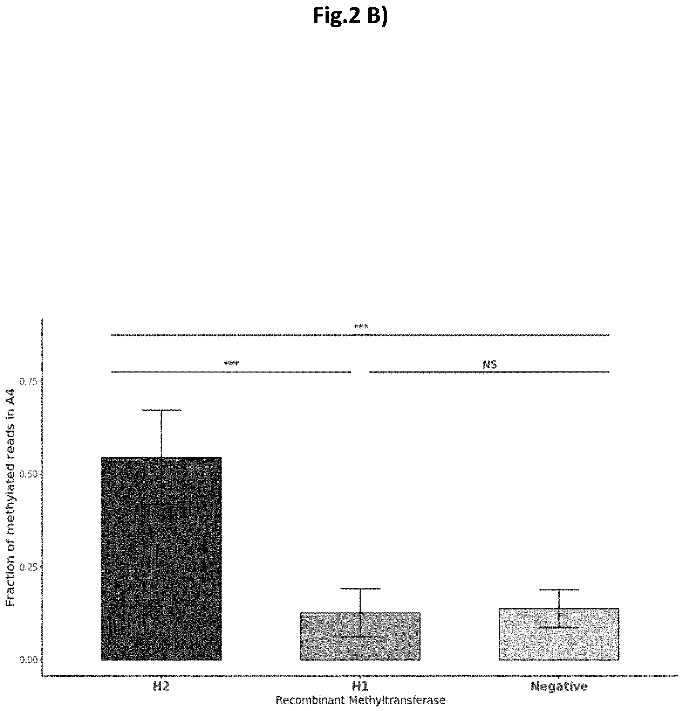

To verify the functionality of the methylase itself the inventors have expressed the methylase recombinantly by shuttling plasmid DNA into H2 and H1 (Fig.2B).

The KO mutant strain was then infected with PAD20 extracted from either H1, A1 or KPA and the activity of the Restriction methylation system measured. This confirmed the hypothesis that epigenetic changes could be involved in phage host range specificity.

To understand if C. *acnes* H2 (KPA171202) is methylating phage DNA during infection phages isolated from H2 were extracted and H2 was also infected with H1 PAD20 phage. Methylation of phage DNA by nanopore sequencing was measured and the specific methylation pattern identified (Fig.2 C).

### Example 3: Specific killing of a population comprising two different C. acnes strains

A strain from subgroup IA1 (A1) was inoculated 1:1 with one of subgroup IB (H1) and this mix was infected with a phage extracted from A1 host. After 3 days a subset of bacteria was plated on agar plates and a subset was checked for classification by SLST sequencing. All tested colonies belonged to H1 C. acnes strain which meant a 100% host range specificity of PAD20 phage. These findings confirm strong selectivity of the tested phage to its host.

### Example 4: Specific killing on a whole microbiome culture extracted from human skin

A diverse bacterial population was inoculated with PAD20 C. *acnes* phage extracted from A1 and samples were taken at 3 different time points to observe specific killing of certain strains in the growing population. Changes depending on strain level but also Clade level were compared. A decrease in Clade IA1, especially A1 strain, was observed, while an increase could be observed in Clade IB. In case of type II strains SLST types K1 and K2 were decreased while other K types stayed unchanged over time. Those seemed to be resistant to phage infection (Fig.3).

### List of References:

1. Chen, Y. et al. Genetic Engineering of Bacteriophages Against Infectious Diseases. Front. Microbiol. 10, 954 (2019).
2. Samson, J. E., Magadán, A. H., Sabri, M. & Moineau, S. Revenge of the phages: defeating bacterial defences. Nat. Rev. Microbiol. 11, 675-687 (2013).
3. Seed, K. D. Battling Phages: How Bacteria Defend against Viral Attack. PLoS Pathog. 11, e1004847 (2015).
4. Furi, L. et al. Methylation Warfare: Interaction of Pneumococcal Bacteriophages with Their Host. J. Bacteriol. 201, (2019).
5. Bryson, A. L. et al. Covalent Modification of Bacteriophage T4 DNA Inhibits CRISPR-Cas9. MBio 6, e00648 (2015).
6. Dreno, B. et al. Cutibacterium acnes(Propionibacterium acnes) and acne vulgaris: a brief look at the latest updates. Journal of the European Academy of Dermatology and Venereology vol. 32 5―14 (2018).
7. Lood, R., Morgelin, M., Holmberg, A., Rasmussen, M. & Collin, M. Inducible Siphoviruses in superficial and deep tissue isolates of Propionibacterium acnes. BMC Microbiol. 8, 139 (2008).
8. Paetzold, B. et al. Skin microbiome modulation induced by probiotic solutions. Microbiome 7, 95 (2019).
9. Madeira, F. et al. The EMBL-EBI search and sequence analysis tools APIs in 2019. Nucleic Acids Res. 47, W636-W641 (2019).
10. Brüggemann, H. et al. The complete genome sequence of Propionibacterium acnes, a commensal of human skin. Science 305, 671-673 (2004).
11. Sorensen, M. et al. Mutagenesis of Propionibacterium acnes and analysis of two CAMP factor knock-out mutants. J. Microbiol. Methods 83, 211-216 (2010).
12. Stoiber, M. et al. De novoldentification of DNA Modifications Enabled by Genome-Guided Nanopore Signal Processing. doi:10.1101/094672.
13. Bailey, T. L., Johnson, J., Grant, C. E. & Noble, W. S. The MEME Suite. Nucleic Acids Res. 43, W39―49 (2015).
14. Kolmogorov, M., Yuan, J., Lin, Y. & Pevzner, P. A. Assembly of long, error-prone reads using repeat graphs. Nat. Biotechnol. 37, 540-546 (2019).
15. Tourancheau, A., Mead, E. A., Zhang, X.-S. & Fang, G. Discovering multiple types of DNA methylation from bacteria and microbiome using nanopore sequencing. Nat. Methods 18, 491―498 (2021).
16. Vasu K, Nagaraja V. Diverse functions of restriction-modification systems in addition to cellular defense. Microbiol MolBiol Rev. 77: 53-72 (2013).

## Claims

1. An *in vivo* or *in vitro* method for screening for changes in the infectivity range of one or more bacteriophages due to the epigenetic imprinting of the bacteriophage(s), the method comprising:
(a) obtaining an isolate of the one or more bacteriophages after infection of at least a bacterium with said one or more bacteriophages, said bacterium preferably comprising a restriction methylation system; and
(b) infecting a bacterium different from the bacterium used in step a), said bacterium preferably comprising a restriction methylation system, with the one or more bacteriophages that previously infected the bacterium of a) in order to allow multiplication of said phage; and
(c) obtaining an isolate of the one or more bacteriophages after infection of step b), and determining the changes in the infectivity range of the one or more bacteriophages due to the epigenetic imprinting of the bacteriophage(s) after the infection of the bacterium of step b); and optionally
(d) selecting said isolate of the one or more bacteriophage if the sought-after specific infectivity range due to the change in the epigenetic imprinting is obtained and, optionally, formulating said bacteriophage(s) into a product.

2. The method of claim 1, wherein the method further comprises characterizing the epigenetic imprinting of the isolate of step c) and/or d).

3. An *in vivo* or *in vitro* method for screening for one or more bacteriophages with a specific infectivity range due to the epigenetic imprinting of the bacteriophage(s), the method comprising:
(a) obtaining an isolate of the one or more bacteriophages after infection of said one or more bacteriophages of at least a bacterium, said bacterium preferably comprising a restriction methylation system; and
(b) infecting a bacterium different from a), said bacterium preferably comprising a restriction methylation system, with the one or more bacteriophages that previously infected the bacterium of a) in order to allow multiplication of said phage; and
(c) obtaining an isolate of the one or more bacteriophages after infection of step b), and determining whether the one or more bacteriophages have the specific infectivity range due to the epigenetic imprinting of the bacteriophage(s) after the infection of the bacterium of step b), and
(d) selecting said isolate of the one or more bacteriophage if the sought-after specific infectivity range due to the change in the epigenetic imprinting is obtained and, optionally, formulating said bacteriophage(s) into a product.

4. The method of any one of claims 1 to 3, wherein the bacterium of step b) comprises a restriction methylation system.

5. The method of any one of claims 1 to 4, wherein the isolate of bacteriophages after infection of step b) comprises a substantially homogenous population of bacteriophages with a changed infectivity range due to the epigenetic imprinting.

6. The method according to any preceding claim, wherein the bacterium of a) and b) is selected from the group consisting of: C. *acnes, E.coli,* Salmonella species, such as *Salmonella typhimurium,* Shigella species, such as *Shigella flexneri,* Enterococcus species, such as *Klebsiella,* Proteobacteria species, such as Pseudomonas species, specifically *Pseudomonas aeruginosa,* Haemophilus species, Firmicutes species, such as Bacillus species, Streptococcus species, such as Streptococcus, and Staphylococcus species, specifically *Staphylococcus aureus,* preferably wherein the bacterium of a) and b) is C. *acnes.*

7. The method according to any preceding claims, wherein each of the bacterium of a) and b) is a different strain of C. *acnes,* and wherein the bacterium of step b) is **characterized by** comprising an active restriction methylation system, such as a type Ig, type Ila, type IIb, type IIg or type IIIb restriction methylation system.

8. The method according to any preceding claims, wherein the bacterium of a) and b) is a different C. *acnes* strain and each of said strains pertains to any of the following clades IA1, IA2, IB, IC, type II or type III.

9. The method according to the preceding claim wherein the bacterium of a) pertains to the IA1, IA2 or IB clade and the bacterium of b) pertains to any of the following clades IA1 or IA2, IB, IC, type II or type III.

10. The method according to the preceding claim wherein the bacterium of a) pertains to the IA1, IA2 or IB clade and the bacterium of b) pertains to any of the following clades IB, IC, type II or type III.

11. A composition comprising a phage population as identified and obtained or as obtainable by the method of any of claims 1 to 10.

12. The composition according to claim 11, for use in therapy.

13. The composition according to claim 11, for use in the selective bacterial killing of one or more bacterial species or strains selected from any species or strains of the group consisting of C. *acnes, E.coli,* Salmonella species, such as *Salmonella typhimurium,* Shigella species, such as *Shigella flexneri,* Enterococcus species, such as *Klebsiella,* Proteobacteria species, such as Pseudomonas species, Haemophilus species, and Firmicutes species, such as Bacillus species, Streptococcus species, such as Streptococcus, and Staphylococcus species, specifically *Staphylococcus aureus,* preferably wherein the bacterium of a) and b) is C. *acnes.*

14. The composition according to claim 11, for use in the selective lysing and/or killing of the one or more bacterial species or strains to enhance engraftment of probiotic strains.

15. Use of the epigenetic imprint which is changed during phage reinfection of a suitable bacterium with a Restriction methylation system to select phages capable of lysing and/or killing one or more bacterial species or strains and to obtain a substantially homogenous population of phages with a specific infectivity range.
